# EUROPÄISCHE PATENTSCHRIFT

(11) **EP 2 854 823 B1**
(45) Veröffentlichungstag und Bekanntmachung des Hinweises auf die Patenterteilung: **06.03.2019**
(21) Anmeldenummer: 13729620.8
(22) Anmeldetag: 31.05.2013
(51) Int. Cl.: A61P 3/12, A61K 33/06, A61K 33/12, A61K 35/02, A61K 45/06, A61P 3/00

(54) **TONMINERAL ZUR REDUZIERUNG VON ANORGANISCHEN PHOSPHATEN, INSBESONDERE IM RAHMEN EINER NIERENERSATZTHERAPIE**
CLAY MINERAL FOR REDUCING INORGANIC PHOSPHATES, IN PARTICULAR IN RENAL REPLACEMENT THERAPY
MINÉRAL ARGILEUX DESTINÉ À RÉDUIRE LES PHOSPHATES INORGANIQUES, EN PARTICULIER DANS LE CADRE D'UNE THÉRAPIE DE SUBSTITUTION RÉNALE

(30) Priorität: 04.06.2012 DE 102012209411
(43) Veröffentlichungstag der Anmeldung: 08.04.2015
(73) Patentinhaber: Fim Biotech GMBH, 10117 Berlin (DE); Fraunhofer-Gesellschaft zur Förderung der angewandten Forschung e.V., 80686 München (DE)
(72) Erfinder: MITZNER, Steffen, 18119 Rostock (DE); KERKHOFF, Claus, 48161 Münster (DE); EMMRICH, Frank, Leipzig 04299 (DE); BREITRÜCK, Anne, 18057 Rostock (DE); BODAMMER, Peggy, 18055 Rostock (DE); KRÜGER, Gerd, 10621 Berlin (DE); DALLWIG, Rainer, 14476 Potsdam (DE)
(74) Vertreter: Maikowski & Ninnemann Patentanwälte Partnerschaft mbB
(86) Internationale Anmeldenummer: PCT/EP2013/061255
(87) Internationale Veröffentlichungsnummer: WO 2013/182485

(56) Entgegenhaltungen:
- EP-A1- 1 270 001
- EP-A1- 2 380 850
- WO-A1-2005/018651
- WO-A1-2009/050468
- WO-A2-2008/013630
- WO-A2-2008/030947
- US-A1- 2010 004 588
- US-A1- 2012 058 157
- DATABASE WPI Week 200903 Thomson Scientific, London, GB; AN 2009-A58254 XP002712471, -& JP 2008 303189 A (MIZUSAWA CHEM IND CO LTD) 18. Dezember 2008 (2008-12-18)
- DATABASE WPI Week 200912 Thomson Scientific, London, GB; AN 2009-E72062 XP002712472, -& CN 101 347 454 A (JINAN KANGZHONG PHARM R & D CO LTD) 21. Januar 2009 (2009-01-21)
- R L HEIN: "Tonerde als Arznei", DIE WELT, 16. Oktober 2008 (2008-10-16), XP055077994,
- C I OBIALO ET AL: "Clay Pica Has No Hematologic or Metabolic Correlate in Chronic Hemodialysis Patients", JOURNAL OF RENAL NUTRITION, Bd. 11, Nr. 1, 1. Januar 2001 (2001-01-01) , Seiten 32-36, XP055077910, DOI: 10.1053/jren.2001

## Beschreibung

Die vorliegende Erfindung betrifft ein silikathaltiges Tonmineral zur Verwendung als Mittel zur Reduzierung der zur Reduzierung der Konzentration von anorganischen Phosphaten in Körperflüssigkeiten und Dialyseflüssigkeiten gemäß Anspruch 1.

### Beschreibung

Phosphate spielen im menschlichen Metabolismus eine bedeutende Rolle und sind für die Funktion des menschlichen Organismus lebensnotwendig. Phosphate werden üblicherweise durch die Nahrung zugeführt und im Darm resorbiert. Bis zu 70% der mit der Nahrung aufgenommenen Phosphate werden über die Niere und den Harn ausgeschieden. Der Rest wird im Organismus verwertet. Bei Menschen mit gesunden Nieren liegt die Menge des im Urin pro Tag ausgeschiedenen Phosphates im Durchschnitt bei 900 mg.

Werden die normalen Phosphatwerte im Blut jedoch überschritten, kommt es aufgrund des niedrigen Löslichkeitsproduktes von Calciumphosphat zu dessen Ablagerung in den Gefäßsystemen und einer damit verbundenen Verkalkung.

Bei einer mangelnden Ausscheidungskapazität der Niere wie z.B. im Falle der Niereninsuffizienz, reichert sich bei den Patienten Phosphat im Organismus in unerwünschter Menge an, so dass es zu einem erhöhten Phosphatspiegel im Blut (Hyperphosphatämie) kommt.

Die chronische Niereninsuffizienz ist gekennzeichnet durch einen langsamen progressiven Verlust der Nierenfunktion. Hauptursachen sind Entzündungen und Infektionen der Nieren, Verengung der ableitenden Harnwege und angeborene Nierenerkrankungen. In den Industrienationen häufen sich jedoch die Diabetes mellitus Typ 2- und die arterielle Hypertonie-vermittelte Niereninsuffizienz. Die Niereninsuffizienz kann nur durch eine Nierenersatztheraphie in Form einer lebenslangen Therapie mit Dialyse oder Nierentransplantation behandelt werden.

Die apparative extrakorporale Blutwäsche ist für Niereninsuffizienz-Patienten eine lebenserhaltende Therapie, die zum Teil über viele Jahre hinweg die exkretorische Funktion der Niere übernehmen muss. In Deutschland werden ca. 78.000 Patienten mit einer Nierenersatztherapie betreut. Weltweit leiden mehr als 5 Millionen Menschen an der chronischen Nierenerkrankung. Trotz stetiger Weiterentwicklung und Verbesserung der Dialysetechnik zeigen Dialysepatienten eine signifikant über dem Niveau der Normalbevölkerung liegende Morbidität- und Mortalitätsrate. Dieses ist begründet in Sekundärkomplikationen in mehreren Organsystemen aufgrund einer fortschreitenden Akkumulation von Urämietoxinen, die für das erhöhte Herz-Kreislauf-Risiko verantwortlich sind.

Aktuell sind 115 Urämietoxine bekannt, wobei Phosphat eines davon ist. Das Phosphat aus der Gruppe der kleinmolekularen wasserlöslichen Urämietoxine unterliegt einer permanenten Kontrolle, da die chronische Niereninsuffizienz zu einer Hyperphosphatämie führt und ein entscheidender Risikofaktor für die Mortalität ist. Eine Reduktion der Phosphatzufuhr soll durch Dialyse, eine phosphatarme Ernährung und eine Einnahme von phosphatbindenden Medikamenten erreicht werden. Die Reduktion der Phosphatkonzentration während der Dialyse im Plasma ist dabei jedoch nur unzureichend, so dass eine weitere Reduktion der exogenen Phosphatzufuhr notwendig ist.

Mittels Phosphatelimination durch phosphatbindende Medikamente sollen die mit der Nahrung aufgenommenen Phosphatmengen im Magen-Darm-Trakt gebunden und am Übertritt in die Blutbahn gehindert werden. Etablierte Therapeutika sind Aluminium- und Kalziumsalze. Sie zeichnen sich jedoch durch erhebliche Nebenwirkungen aus. So führen Ablagerungen von Aluminium im Skelettsystem und Gehirn zu schweren Beeinträchtigungen der Hämatopoese und von zerebralen Funktionen. Kalziumsalze sind aufgrund einer Entwicklung einer Hyperkalzämie und dem Anstieg des Kalziumphosphats von großem Nachteil. Sevelamer und Lathancarbonat (Fosrenol) sind neuere kalzium- und aluminiumfreie Phosphatbinder. Zu den häufigsten Nebenwirkungen aller vier Phosphatbinder zählen Beschwerden des Gastrointestinaltrakts. So kann z.B. Fosrenol, wie auch die anderen klinisch eingesetzten Phosphatbinder Magen-Darm-Störungen, wie Übelkeit, Erbrechen, Durchfall, Verstopfung, Abdominalschmerzen, Kopfschmerzen, Krampfanfälle und Enzephalopathie. Lanthan besitzt zudem eine lange Halbwertszeit und reichert sich in Knochen und verschiedenen Geweben, wie Zähne, Leber, Nieren oder Gehirn an.

Zudem ist die medikamentöse Therapie dauerhaft und stellt somit eine erhebliche Kostenbelastung für das Gesundheitssystem dar.

Aus der WO 2009/050468 A1 ist die Verwendung von gemischten Metallverbindungen als Phosphatbinder bekannt. Hierbei handelt es sich bevorzugt um Substanzen enthaltend zwei- und dreiwertige Metallionen. Als zweiwertige Metallkationen werden dabei Mg2+, Zn2+, Fe2+, Cu2+, Ca2+, La2+, Ce2+und Ni2+ benannt. Die dreiwertigen Metallkationen können unter anderem Mn3+, Fe3+, La3+ und Ce3+ sein Derartige Zusammensetzungen weisen eine "Layered Double Hydroxide" Struktur auf und werden auch als anionische Tonmineralien bezeichnet.

Der vorliegenden Erfindung liegt daher das Problem zu Grunde, eine Verbindung bzw. Zusammensetzung zur Reduktion der exogenen Phosphatzufuhr oder zu Reduzierung des Phosphatsgehaltes im Plasma zur Verfügung zu stellen, welche die oben genannten Nachteile nicht aufweist.

Diese Aufgabe wird durch ein silikathaltiges Tonmineral mit den Merkmalen des Anspruchs 1 gelöst.

Entsprechend wird ein silikathaltiges Tonmineral zur Verwendung als Mittel zur Reduzierung der Konzentration von anorganischem Phosphat in Körperflüssigkeiten oder Dialyseflüssigkeiten zur Behandlung von Hyperphosphatämie bereitgestelllt. . Als Körperflüssigkeiten werden insbesondere Blut, Plasma und/oder die Darminhaltflüssigkeit von Menschen angesehen.

Das zum Einsatz kommende silikahaltige Tonmineral weist eine mittlere Teilchengröße von 0,1 bis 1,5 µm auf und wird erfindungsgemäß vor der Verwendung bei einer Temperatur zwischen 400°C und 800°C, bevorzugt zwischen 500°C und 700°C, insbesondere bevorzugt bei 550°C über einen Zeitraum von 60 min bis 240 min, bevorzugt 90 min bis 180 min, insbesondere bevorzugt 120 min thermisch behandelt ..

Die thermische Behandlung des silikathaltigen Tonminerals vor dessen Verwendung bewirkt eine vollständige Dehydrierung und eine Beseitigung von im Tonmineral ggf. enthaltenden organischen Kohlenstoff und weiterer organischer Stoffe. Das thermisch behandelte Tonmineral weist überraschenderweise eine gegenüber dem unbehandelten Tonmineral verbesserte Phosphatbindekapazität auf. Auch weist das thermisch behandelte Tonmineral im Vergleich zu den bisher typischerweise verwendeten Phosphatbindern eine erhöhte bzw. eine gleichwertige Phosphatbindungskapazität auf ohne allerdings deren unerwünschte Nebenwirkungen, insbesondere bei einer oralen Verabreichung.

Das silikathaltige Tonmineral ist zur Reduzierung der Konzentration von anorganischem Phosphat, insbesondere im Blutplasma, zur Behandlung der Hyperphosphatämie, insbesondere im Rahmen einer Nierenersatztherapie geeignet. Das Tonmineral kann demnach zur Herstellung einer Zusammensetzung zur Behandlung von Hyperphosphatämie verwendet werden.

Somit wird vorliegend ein silikathaltiges Tonmineral als Phosphat-Adsorber verwendet. Diese Mineralien bieten aufgrund spezifischer Eigenschaften wie Quellvermögen, Ionenaustauschfähigkeit, Fähigkeit zur Thixotropie und Adsorption von Mykotoxinen oder Lipopolysacchariden (LPS) vielfältigste Einsatzmöglichkeiten bei Mensch und Tier.

Entsprechend der vorliegenden Erfindung ist es demnach vorgesehen, das silikathaltige Tonmineral entweder oral zur Reduzierung der exogenen Phosphatzufuhr einzunehmen, als auch extern, d.h. heißt ohne orale Einnahme, unmittelbar in der Dialyseflüssigkeit während des Dialysevorganges zur Reduzierung des Phosphatgehaltes einzusetzen. Im Falle einer oralen Einnahme kann das Tonmineral auch ohne thermische Behandlung, d.h. in seiner Ausgangsform verwendet werden.

In einer weiteren bevorzugten Ausführungsform wird das silikathaltige Tonmineral vor seiner Verwendung nach der thermischen Behandlung mit zweiwertigen Kationen, insbesondere Magnesium-Ionen angereichert. Die Anreicherung des thermisch behandelten Tonminerals mit zweiwertigen Kationen, wie z.B. Magnesium-Ionen führt zu einer nochmals verbesserten Phosphatbindungskapazität des Tonminerals. Prinzipiell sind alle zweiwertigen Kationen vorliegend einsetzbar, wobei lediglich auf die Verträglichkeit der zweiwertigen Kationen zu achten ist. So ist es vorstellbar anstatt oder zusätzlich zu den genannten Mg²⁺ -Ionen auch Ca²⁺ oder Fe²⁺ -Ionen zu verwenden.

Der Gehalt an zweiwertigem Kation wie Mg²⁺ im silikathaltigen Tonmineral kann in einem Bereich zwischen 5 und 20 Masseanteil% (bezogen auf das trockene Tonmineral), bevorzugt zwischen 5 und 15 Masseanteil%, insbesondere bevorzugt bei 10 Masseanteil% liegen. So weist z.B. 1 g mit Mg²⁺ adsorbiertes Tonmineral 100 mg Mg²⁺ auf.

Die Anreicherung des silikathaltigen Tonminerals mit zweiwertigen Kationen, wie den Magnesium-Ionen wird bevorzugt unter Verwendung von einem Magnesiumsalz, insbesondere Magnesiumchlorid oder Magnesiumsulfat, mit einem Massenanteil zwischen 30 und 70%, bevorzugt 45 und 60% durchgeführt.

In einer Ausführungsform wird die zum Einsatz kommende Magnesiumionen-haltige Lösung in einem Diaphragmalyseverfahren hergestellt. Dabei wird durch eine Elektrolyse eine Magnesiumchloridlösung in ein Anolyt (Chlorwasser) und in ein Katholyt (Magnesiumwasser) aufgespalten. Ein Diaphragma trennt beide Wässer voneinander. Das Anolyt ist sehr sauer und oxidiert, das Katholyt sehr basisch und reduziert. Die Magnesiumlösung hat bevorzugt einen pH-Wert zwischen 7-10, insbesondere bei ca. 9.

In einer weiteren bevorzugten Ausführungsform weist das thermisch behandelte und mit Magnesium-Ionen angereicherte Tonmineral eine mittleren Teilchengröße von 0,1 bis 1 µm, bevorzugt von 0,3 bis 0,8 µm, insbesondere bevorzugt von 0,5 µm auf. Die Vermahlung des Tonminerals kann z.B. einer Rührwerkskugelmühle vorgenommen werden.

Die Phosphatbindungskapazität des so modifizierten silikathaltigen Tonminerals, d.h. des thermisch behandelten, mit Magnesium-Ionen angereicherten und zu einer mittleren Teilchengröße von 0,4-0,6 µm vermahlende Tonminerals, liegt um ein wesentliches über der Phosphatbindekapazität des unbehandelten Tonminerals (ca. um den Faktor 600) und liegt somit sogar über der Phosphatbindekapazität der bisher bekannten Phosphatbinder wie z.B. Fosrenol ohne deren Nachteile wie Magen-Darm-Unverträglichkeiten aufzuweisen.

Mineralien kann man generell in 10 unterschiedliche Klassen einteilen, wobei jede Klasse für sich wieder in mehreren verschiedenen Unterklassen aufgeteilt werden kann. Viele Mineralien haben ihre bevorzugten Einsatzgebiete. Für adsorptive Prozesse kommen insbesondere aus der Silikatgruppe das Gerüstsilikat Zeolith und das Schichtsilikat Montmorillonit zum Einsatz. Zur besseren Vergleichbarkeit der verschiedenen Montmorillonitqualitäten wurde der Begriff Bentonit geprägt, dessen mineralogische Zusammensetzung mindestens 60 bis 70% Montmorillonit enthalten muss. Für viele technische Anwendungen ist diese Gruppenbildung sehr hilfreich. Die Wirkung von Mineralen in biologischen Systemen geht aber weit über Adsorptionsprozessen hinaus. Deshalb ist die alleinige Fixierung auf nur einen möglichst hohen Montmorillonitanteil in der mineralischen Verbindung nicht zielführend.

Die häufig in der Natur vorkommenden mineralischen Verbindungen bzw. Matrizen, mit Wechsellagerungstonminerale (Mixedlayer) als Hauptbestandteil, werden auf Grund ihrer im Vergleich zu reinen Montmorillonitschichten geringeren messbaren spezifischen Oberflächen, Quellvermögen und Kationenaustauschkapazitäten seltener für adsorptive und katalytische Applikationen verwandt.

Es hat sich nunmehr herausgestellt, dass natürlich vorkommende mineralische Verbindungen bzw. Matrizen mariner Genese aus Mixedlayer oder Wechsellagerungsmineralien bestehen, die sich aus quellfähigen und nichtquellfähigen Schichten in unregelmäßiger Folge zusammensetzen und die ggf. noch anderen Minerale wie Silikate, Oxide, Carbonate, Sulfide und Sulfate enthalten, nach einer entsprechenden Aufbereitung durchaus adsorptive und andere interessante Eigenschaften aufweisen.

Mixedlayer können strukturell aus sehr unterschiedlichen Wechsellagerungsschichten bestehen z.B. Kaolinit/Smectit, Chlorit/Vermikulit, Glimmer/Vermikulit oder sehr häufig Wechsellagerung von Illit/Smectit oder Illit/Montmorillonit. Dadurch sind vielfältigere Austauschreaktionen von Kationen und Anionen möglich als bei reinen Montmorilloniten. Deshalb sind Mixedlayer in einer Kombination mit anderen reaktiven Mineralen besonders gut geeignet für die Bindung von in Lösung befindlichen Stoffen in biologischen Systemen.

In einer Ausführungsform der vorliegenden Erfindung wird ein silikathaltiges Tonmineral verwendet, das mindestens ein Wechsellagerungstonmineral umfasst.

Besonders bevorzugt kommt dabei ein Wechsellagerungstonmineral aus Montmorillonit und Illit/Muskovit zum Einsatz, wobei in diesem Wechsellagerungsmineral Montmorillonit und Illit/Muskovit in einem Verhältnis von 60:40 bis 40:60 enthalten sein können, wobei ein Verhältnis von 50:50 bevorzugt ist. Dass heißt, das Montmorillonit und Illit/Muskovit zu jeweils 50 Gew% enthalten sein können.

Zusätzlich zu den Mineralien Montmorillonit und Illit/Muskovit kann das bevorzugt verwendete Tonmineral auch Anteile von anderen Tonmineralien, wie Kaolinite und Chlorite, Carbonate, Sulfide, Oxide und Sulfate aufweisen.

In einer bevorzugten Ausführungsform weist das silikathaltige Tonmineral ein Fe²⁺ / Fe³⁺ - Verhältnis zwischen 0,3 und 1,0, bevorzugt 0,45 und 1,0 auf. So ist in der vorliegenden mineralischen Verbindung das Fe²⁺ / Fe³⁺ - Verhältnis um das ca. 10fache höher als in anderen bekannten Tonmineralien und weist aufgrund dieses erhöhten Fe²⁺ / Fe³⁺ - Verhältnisses ein hohes natürliches antioxidatives Potential auf.

In einer besonders bevorzugten Ausführungsform umfasst das vorliegende silikathaltige Tonmineral durchschnittlich 50-60 Gew%, bevorzugt 55 Gew% Montmorillonit-Muskovit-Wechsellagerung, 15-25 Gew%, bevorzugt 20 Gew % Illit, 5-9 Gew%, bevorzugt 5 Gew% Kaolinit/Chlorit, 10-20 Gew%, bevorzugt 15 Gew% Quarz, 1-2 Gew%, bevorzugt 1 Gew% Calcit, 0,9-1,5, bevorzugt 1 Gew% Dolomit, 0,9-1,9 Gew%, bevorzugt 1 Gew% Feldspat, 0,9 - 1,0 Gew%, bevorzugt 1 Gew% Pyrit und 0,6-1,0 Gew%, bevorzugt 1 Gew% Gips.

Die chemische Zusammensetzung der Hauptelemente kann in Gew% wie folgt angegeben werden: SiO₂ 57,9-59,5; Al₂O₃ 17,0-18,5; Fe₂O₃ 5,9-7,0; K₂O 2,8-3,5; MgO 1,5-2,6; Na₂O 0,9-1,5; TiO₂ 0,6-1,5; CaO 0,25-0,35; P₂O₅ 0,09-0,15; Sonstige 8,9-10,5.

Ein Teil der im unbehandelten Ausgangstonmineral enthaltenen Minerale geht bei Vorhandensein eines Lösungsmittels in dieses über. So ergab ein Suspensionsversuch mit 2 Gew% mineralischer Verbindung in destilliertem Wasser, wobei die Suspension mittels Ultrazentrifuge getrennt und dann der Überstand analysiert wurde, folgende Ergebnisse: Leitfähigkeit 346 µS; pH-Wert 7,3; Kalium 5 mg/l; Natrium 73 mg/l; Chlor 20 mg/l; Magnesium 0 mg/l; Kalzium 1 mg/l; SO₄ 121 mg/l; Aluminium 0 mg/l; SiO₂ 8 mg/l; Eisen 0 mg/l. Besonders hervorzuheben ist der hohe Anteil an Sulfaten und die Tatsache, dass keine Aluminium-Ionen in Lösung gehen. Dies ist besonders für die humane Anwendung von besonderer Bedeutung.

Eine weitere Besonderheit des unbehandelten Ausgangstonminerals ist der hohe Anteil an organischem Kohlenstoff von 0,4 Gew%. Zusammen mit den vorhandenen Sulfaten und Sulfiden ist dies ein wichtiger Beweis für die marine Genese der mineralischen Verbindung und gleichzeitig ein wesentlicher Beitrag zur Wirksamkeit derselben. So weist die mineralische Verbindung bevorzugt mindestens ein Eisensulfid, insbesondere amorphes Pyrit FeS₂ auf, wobei das Eisensulfid in einem Massenanteil zwischen 0,5 % bis 5 %, bevorzugt 0,9 % bis 3,0 % in der mineralischen Verbindung vorhanden sein kann.

Das unbehandelte Tonmineral weist eine BET-Oberfläche von 50 -100 m²/g, bevorzugt 55 - 65 m²/g, insbesondere bevorzugt von 60 m²/g auf. Die innere Oberfläche der bevorzugt verwendeten mineralischen Verbindung ist somit z.B. im Vergleich zu den hochquellfähigen Montmorilloniten relativ gering.

Die BET-Oberfläche des Tonminerals konnte durch die einzelnen Aufbereitungsschritte erheblich vergrößert werden. So weist das thermisch behandelte, mit zweiwertigen Kationen angereicherte und zu einer Teilchengröße zwischen 0,1 bis 1,5 µ vermahlende Tonmineral eine BET-Oberfläche von 200 bis 600 m²/g, bevorzugt 300 bis 500 m²/g, insbesondere bevorzugt von 400 bis 450 m²/g auf.

Das bevorzugt verwendete unbehandelte Ausgangstonmineral wird aus den in Deutschland in Mecklenburg-Vorpommern, genauer in der Nähe von Friedland im östlichen Teil der Mecklenburgischen Seenplatte, vorhandenen Tonvorkommen isoliert und aufbereitet.

Die bevorzugt verwendete mineralische Verbindung weist, wie bereits oben erwähnt, einzigartige Eigenschaften auf, in denen es sich von anderen mineralischen Matrizen wie Bentonit oder Montmorillonit wesentlich unterscheidet.

So ist die vorliegend verwendete mineralische Verbindung nicht nur in der Lage, Kationen in den vorhandenen Montmorillonitschichten auszutauschen, sondern auch Anionen zu binden. Der Wirkmechanismus der vorliegend zum Einsatz kommenden mineralischen Verbindung ist daher ein anderer als bei Bentoniten. So werden z.B. anionische Stoffe besonders gut an den Bruchkanten der in der verwendeten mineralischen Verbindung enthaltenen Illite/Muskovite locker gebunden. Ursache dafür sind fehlende bzw. herausgeschlagene Kaliumionen, die im Mineralverbund für einen Ladungsausgleich sorgen. So entstehen positive Ladungen, an denen Anionen locker gebunden werden können. Da bioaktive Anionen eine relativ hohe Molekularmasse und damit Durchmesser aufweisen, sind die an den Bruchkanten entstandenen Vertiefungen nicht groß genug, um eine feste Bindung zu realisieren. Dies gelingt nur mit kleineren Molekülen, wie die sehr hohe Adsorptionskraft für insbesondere sauerstoffhaltige Molekülanionen wie Phosphat PO₄³⁻, Nitrat NO₃⁻, Nitrit NO₂⁻ und andere beweist. Hier spielen auch die Fe²⁺ -Ionen an den Bruchkanten und hydrierte Eisenoxide (FeO(OH)) aus der Pyritoxidation eine entscheidende Rolle, da diese als Gegenionen fungieren können.

Um eine möglichst hohe Bindungskapazität der vorliegenden mineralischen Verbindung zu erreichen, ist eine Feinstzerkleinerung zur Schaffung einer möglichst hohen Anzahl von Bruchkanten vorteilhaft. Wie bereits oben erwähnt liegt die Teilchengröße dabei bei ca. 0,1 bis 1,5 µm, insbesondere 0,3 bis 0,8 µm, ganz besonders bevorzugt bei 0,4 bis 0,5 µm.

Zudem hat sich im Rahmen von Zetapotentialuntersuchungen herausgestellt, dass durch die Feinstzerkleinerung z.B. mittels einer Gegenstrahlmühle sich das Zetapotential der mineralischen Verbindung bei pH 7 auf einen Wert zwischen 10 und 100 mV, bevorzugt 30 und 90 mV, insbesondere bevorzugt 45 und 90 mV einstellt, was auf eine starke Zunahme der positiven Andockstellen für Anionen hindeutet.

Das vorliegend verwendete silikathaltige Tonmineral wird bevorzugt in einem Verfahren mit den folgenden Schritten gewonnen:
a) Zerkleinerung des unbehandelten Tonminerals auf eine mittlere Teilchengröße zwischen 1 und 3 µm, bevorzugt 1 und 2 µm, insbesondere bevorzugt 1,2 und 1,5 µm und Trocknen auf eine Endfeuchte zwischen 0,05 und 1 Ma%, bevorzugt 0,1 und 0,5 Ma%, insbesondere bevorzugt 0,1 Ma und 0,2 Ma%; und
b) thermische Behandlung des zerkleinerten Tonminerals bei einer Temperatur zwischen 400°C und 800°C, bevorzugt zwischen 500°C und 700°C, insbesondere bevorzugt bei 550°C über einen Zeitraum von 60 min bis 240 min, bevorzugt 90 min bis 180 min, insbesondere bevorzugt 120 min.

Das Ausgangstonmineral wird aus Rohton in Form von Tonpellets mit einer Größe von 10 bis 50 mm, bevorzugt 15 bis 30 mm und einer Feuchte von 10 bis 50 Masseanteil%, bevorzugt 15 bis 30 Masseanteil% gewonnen. Die mittlere Teilchengröße der Tonteilchen in den Tonpellets beträgt 5 bis 15 µm, bevorzugt 7 bis 12 µm, insbesondere 9µm.

Das anfängliche Zerkleinern des Ausgangstonminerals gemäß Schritt a) erfolgt z.B. in einer dreistufigen Prallmühlen-Sichter-Kombination zur gleichzeitigen Zerkleinerung und schonenden Trocknung des Ausgangstonminerals auf die oben genannte Teilchengröße und Endfeuchte. In diesem Verfahrensschritt erfolgt eine Anreicherung der Tonmineralien durch Abtrennung von funktionslosen Mineralien um bis zu 50% und eine Erhöhung des Wasseraufnahmevermögens um bis zu 90%.

In der sich in Schritt b) anschließenden thermischen Behandlung des zerkleinerten Ausgangstonminerals findet eine Thermoaktivierung des Tonminerals bei gleichzeitiger vollständiger Dehydration und Beseitigung von organischen Stoffen aus dem Tonmineral statt.

In einem anschließenden Verfahrensschritt c) wird das thermisch behandelte Tonmineral in einer zweiwertige Kationen-, insbesondere Magnesiumionen- enthaltenden Lösung unter Ausbildung einer kolloidalen Lösung dispergiert. Der pH-Wert der verwendeten Magnesiumionen-Lösung beträgt 7-10, bevorzugt 9. Die Konzentration der Lösung an zweiwertigen Kationen, wie Mg²⁺ -Ionen beträgt bevorzugt 5 bis 10 Ma%, insbesondere 7 Ma%. Der Anteil an zweiwertigen Kationen wie Mg²⁺ im behandelten Tonmineral beträgt dann bevorzugt 10 Ma% bezogen auf die Tonmineraltrockenmasse, wie oben bereits ausgeführt.

Die Menge des Tonminerals, das in der Magnesiumionen-enthaltenden Lösung dispergiert wird, kann bei 10 bis 50 Masseanteil%, bevorzugt 10 bis 30 Masseanteil%, insbesondere bevorzugt bei 15 bis 20 Masseanteil% liegen.

In einem weiteren Schritt d) wird die kolloidale Mg-Tonmineral-Lösung bevorzugt auf eine mittlere Teilchengröße von 0,1 bis 1,5 µm, bevorzugt von 0,3 bis 0,8 µm, insbesondere bevorzugt von 0,4 bis 0,5 µm vermahlen. Das Vermahlen des kolloidalen Tonminerals kann z.B. unter Verwendung einer Rührwerkskugelmühle erfolgen. Während der Vermahlung kommt es zu einer weitergehenden Einarbeitung der Magnesium-Ionen in die mineralische Matrix.

Im Anschluss an die Kolloidvermahlung des thermisch behandelten und mit zweiwertigen Kationen, insbesondere Magnesium-Ionen angereicherten Tonminerals kann die kolloidale Tondispersion mittels vorhandener Trocknungsverfahren, insbesondere durch ein VakuumKontakt-Trocknungsverfahren z.B. auf eine Restfeuchte von 1 bis 10 Masseanteil%, bevorzugt 3 bis 8 Masseanteil%, insbesondere bevorzugt 4-5 Masseanteil% getrocknet werden.

Das vorliegende silikathaltige Tonmineral wird demnach in bevorzugter Weise in Form einer sterilisierten Mineralsuspension verwendet, die z.B. mittels Autoklavierung sterilisierbar ist. Das Tonmineral kann auch in einer kolloid dispersen und/oder gelartigen Form verwendet werden, wobei eine 15 % Suspension besonders bevorzugt ist.

Das vorliegende silikathaltige Tonmineral kann in Form einer Kapsel bevorzugt in einer Einmaldosis eingenommen werden. Es ist vorstellbar, dass das Tonmineral als Einmaldosis bis zu 5 x täglich verabreicht werden kann. Die Tagesgesamtmenge des aufgenommenen Tonminerals sollte bevorzugt zwischen 1 g bis 50 g, bevorzugt 5 g bis 30 g, insbesondere 5 g bis 15 g betragen. Entsprechend kann die Menge einer Einzeldosis Tonmineral bei einer Tagesgesamtmenge von 5-15 g 1-3 g betragen. Die Wahl der zu verabreichenden Menge an Tonmineral wird von einer Vielzahl von Faktoren beeinflusst und ist entsprechend individuell anzupassen.

Es ist aber auch möglich das silikathaltige Tonmineral in Form einer Suspension z.B. 15%-30% Suspension zu verabreichen. Eine Variante wäre z.B. 5ml aus einem Quetschschlauch bis zu 3 x täglich 1 h nach der Mahlzeit. Auch hier ist eine individuelle Anpassung der zu verabreichenden Menge an Tonmineral erforderlich.

Das vorliegende silikathaltige Tonmineral weist je nach Behandlungsschritt unterschiedliche maximale Phosphatbindungskapazitäten Qmax [mg/g] auf. Qmax als maximale Phosphatbindekapazität wird aus dem Langmuir-Isothermen-Modell berechnet. Es ist ein theoretischer Wert, der sich bei einer hohen Phosphatbelastung pro Menge Adsorber ergibt.

Das unbehandelte Ausgangstonmineral ist z.B. durch eine maximale Phosphatbindungskapazität von ca. 0,2 mg/g gekennzeichnet, die durch die verschiedenen Behandlungsschritte zum Teil um ein Vielfaches gesteigert werden kann.

So weist das lediglich gemäß Verfahrensschritt a) zerkleinerte Tonmineral bereits eine maximale Phosphatbindungskapazität von 2 bis 4 mg/g, bevorzug 3 mg/g auf, wohingegen das nach Schritt b) thermisch behandelte Tonmineral bereits eine maximale Phosphatbindungskapazität von 10-30 mg/g, bevorzugt 20 mg/g aufweist. Die Anreicherung des thermisch behandelten Tonminerals mit zweiwertigen Kationen, wie z.B. Magnesium-Ionen bewirkt einen weiteren leichten Anstieg der maximalen Phosphatbindungskapazität auf 20 bis 40 mg/g, bevorzugt 30 mg/g.

Nach Kolloidvermahlung des thermisch behandelten und mit zweiwertigen Kationen, insbesondere Mg²⁺⁻Ionen, behandelten Tonminerals gemäß Schritt d) erhöht sich die maximale Phosphatbindungskapazität auf Werte zwischen 100 bis 150 mg/g, bevorzugt 120 mg/g.

Die vorliegende Erfindung wird anhand der folgenden Ausführungsbeispiele unter Bezugnahme auf die Figuren näher erläutert. Es zeigen:
- Fig. 1: ein erstes Diagramm zum Nachweis der Phosphatbindungskapazität des vorliegend behandelten Tonminerals;

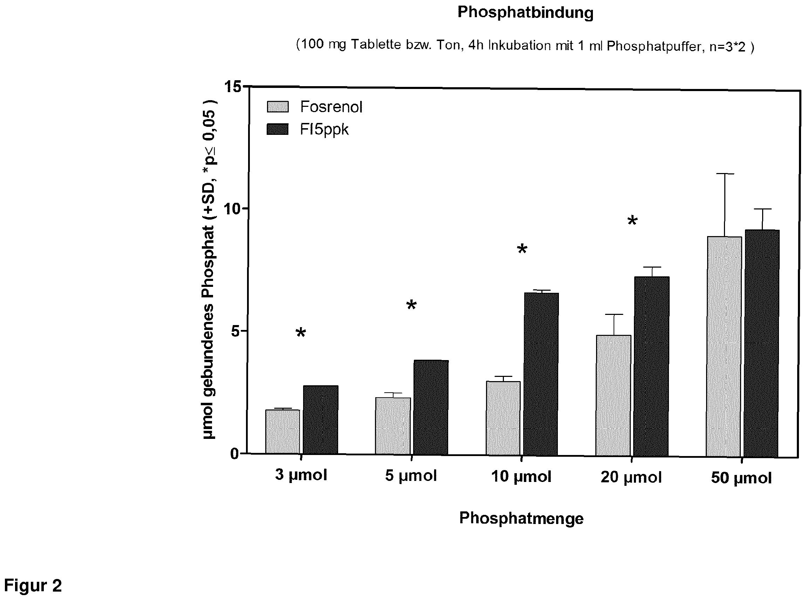

- Fig. 2: ein zweites Diagramm zum Nachweis der Phosphatbindungskapazität des vorliegend verwendeten thermisch behandelten Tonminerals in Abhängigkeit von der vorhandenen Phosphatmenge; und
- Fig. 3: ein drittes Diagramm zum Vergleich der Phosphatbindungskapazität des vorliegend thermisch behandelten Tonminerals und klinisch verwendeter Phosphatbinder.

Vorliegend wurde ein Muskovit-Montmorillonit-Illit-Wechsellagerungsmineral auf seine Eigenschaften als Phosphatbinder untersucht, wobei das Tonmineral verschiedenen Behandlungsschritten ausgesetzt wurde.

Das Friedländer Muskovit-Montmorillonit-Illit-Wechsellagerungsmineral zeichnet sich durch eine geringe Teilchengröße mit einer großen Oberfläche und quellfähige Einzelschichten aus Natrium-Montmorillonit aus, die dem Silikat ein hohes Wasseraufnahmevermögen verleihen. Gleichzeitig können die in den einzelnen Zwischenschichten vorhandenen Elemente leicht gegen anorganische und organische Stoffe ausgetauscht werden. Dieser Vorgang ist reversibel und für das Adsorptionsverhalten des Friedländer Wechsellagerungsmineral von zentraler Bedeutung. Ein wesentlicher Vorteil des Friedländer Silikats gegenüber anderen Silikaten aus anderen weltweiten Lagerstätten ist die geringe Freisetzung von Aluminiumionen. Dies konnte in einer Studie der Bundesanstalt für Geowissenschaften und Rohstoffen nachgewiesen werden. Eine Ursache hierfür ist der Aufbereitungsprozess, der ohne eine Säureaktivierung der Tonminerale auskommt und damit keine Aluminiumionen freisetzt. Erst die schonende Aufbereitung der mineralischen Verbindung ermöglicht die Nutzung des Friedländer Silikats für gesundheitliche Zwecke.

In den folgenden Untersuchungen wurde die Phosphat-Bindekapazität des Friedländer Tonminerals nachgewiesen, welcher vorher verschiedene Behandlungsprozess unterzogen wurde:
- Fl15TP: unbehandeltes Ausgangstonmineral mit einer Feuchte von 15-25Ma% und einer Teilchengröße von ca. 9µm
- Fl5pp: auf eine mittlere Teilchengröße von 1,4 µm zerkleinertes Tonmineral mit einer Endfeuchte von 0,1 Ma%;
- Fl5ppK: thermoaktiviertes Fl5pp (thermische Behandlung bei 550°C für 120 min),
- Fl5ppK-D-MG: Dispergierung des Fl5ppK mit 15-20Ma% in einem Magnesium-Katolyth-Wasser (pH 9), das mit einem Diaphragmalyse-Verfahren hergestellt wurde; und
- Fl0,5ppK-D-MG-KV: Kolloidvermahlung des Fl5ppK-D-Mg mittels einer Rührwerkskugelmühle auf eine mittlere Teilchengröße von 0,5 µm.

Zur Messung der Phosphatbindungskapazität wurden die verschieden behandelten Proben des Friedländer Tonminerals mit einer Phosphat-Lösung für eine Stunde inkubiert. Der gewonnene Überstand konnte anschließend am Analysengerät Cobas Mira hinsichtlich seiner Phosphatkonzentration untersucht werden.

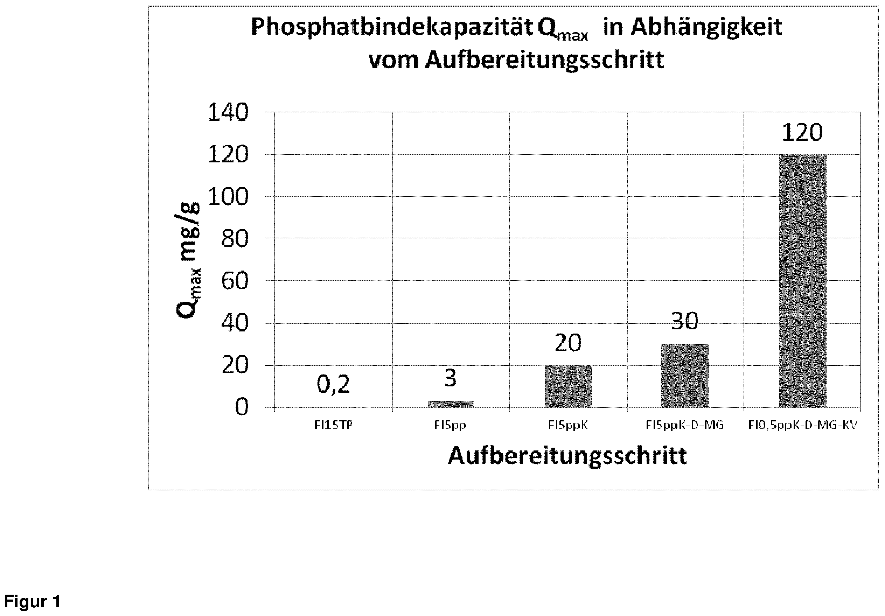

Die Ergebnisse zeigen eine von der Vorbehandlung des Tonminerals abhängige maximale Phosphat-Bindekapazität des Friedländer Tonminerals (siehe Figur 1). So beträgt die maximale Phosphatbindekapazität Qmax von FI15TP 0,2 mg/g, von Fl5pp 3 mg/g, Fl5ppK 20 mg/g, Fl5ppk-D-MG 30 mg/g und Fl0,5ppK-D-Mg-KV 120 mg/g.

Die maximale Phosphatbindungskapazität ist stark von der vorhandenen und zu bindenden Phosphatmenge abhängig. Die Phosphatbindung wurde nach 4h Inkubation mit 1 ml Phosphatpuffer bestimmt. So zeigt sich für Fl5ppK eine steigende Phosphatbindung mit steigender Phosphatmenge, jedoch erfolgt dieser Anstieg nicht linear. Dasselbe ist zutreffend für das Vergleichspräperat Fosrenol. Interessanteweise liegt allerdings die Phosphatbindungskapazität des Fl5ppK bei niedrigeren Phosphatmengen über der Bindungskapazität des Fosrenols und gleicht sich erst bei einer Phosphatmenge von 50 µmol aneinander an.

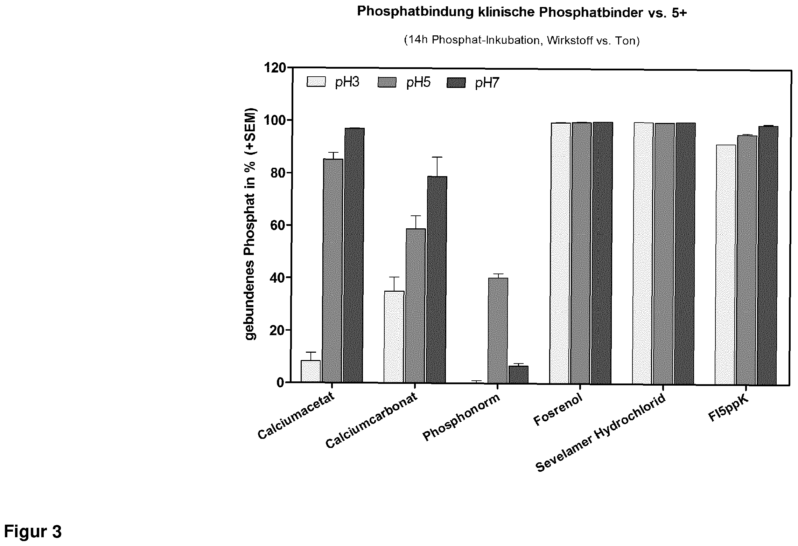

In den weiteren in Figur 3 gezeigten Untersuchungen wurde die Phosphat- Bindekapazität des Fl5ppK mit 5 klinisch eingesetzten Phosphatbindern bei verschiedenen pH-Werten verglichen: die kalziumhaltigen Phosphatbinder Calcium-acetat und Calcium-carbonat, Phosphonorm, der kalzium- und aluminiumfreie Phosphatbinder Fosrenol und Sevelamer Hydrochlorid.

Das Diagramm der Figur 3 zeigt das gebundene Phosphat in % nach einer 14 stündigen Inkubation bei den pH-Werten 3, 5 und 7.

Die Phosphatbinder Calcium-acetat, Calcium-carbonat und Phosphonorm weisen eine starke pH-Abhängigkeit der Phosphatbindungskapazität auf, während Phosphatbinder Fosrenol, Sevelamer Hydrochlorid und das erfindungsgemäße Fl5ppK nicht pH-Wert abhängig sind. Die Phosphatbindungskapazität des Fl5ppK liegt bei den pH-Werten 3, 5 und 7 auf dem gleichen Niveau wie Phosphatbinder Fosrenol und Sevelamer Hydrochlorid.

Die Untersuchungen zeigen, dass das thermisch behandelte Friedländer Tonmineral Fl5ppK eine hohe Phosphat- Bindekapazität aufweisen, welche unabhängig vom pH-Bereich ist.

Auch der Vergleich mit gängigen klinischen Phosphatbindern zeigt ein ähnliches Phosphat-Bindepotenzial.

Die vorliegenden Untersuchungen zeigen somit, dass das Friedländer Tonmineral, insbesondere in seinen behandelten Formen, sich durch hohe, im pH-Bereich von 3-8 stabile Phosphat-Bindekapazitäten auszeichnet. Der Vergleich mit klinisch-angewandten Phosphatbindern ergab, dass der behandelte Friedländer Tonmineral eine auf die Wirkstoffmenge bezogene vergleichbare Phosphat-Bindekapazität besitzt. Das Friedländer Tonmineral zeigt zudem nur eine geringe Freisetzung von Aluminiumionen.

Das Friedländer Tonmineral, insbesondere in seinen behandelten Modifikationen, eignet sich als Phosphatbinder und kann zur Reduktion der exogenen Phosphatzufahr aus der Nahrung bei Niereninsuffizienz-Patienten eingesetzt werden.

## Patentansprüche

1. Silikathaltiges Tonmineral zur Verwendung als Mittel zur Reduzierung der Konzentration von anorganischem Phosphat in Körperflüssigkeiten oder Dialyseflüssigkeiten zur Behandlung von Hyperphosphatämie,
**dadurch gekennzeichnet,**
**dass** das silikathaltige Tonmineral eine mittlere Teilchengröße von 0,1 bis 1,5 µm aufweist, und dass das Tonmineral vor der Verwendung thermisch bei einer Temperatur zwischen 400°C und 800°C, bevorzugt zwischen 500°C und 700°C, insbesondere bevorzugt bei 550°C über einen Zeitraum von 60 min bis 240 min, bevorzugt 90 min bis 180 min, insbesondere bevorzugt 120 min behandelt wird.

2. Silikathaltiges Tonmineral zur Verwendung nach Anspruch 1 zur Reduzierung der Konzentration von anorganischem Phosphat im Blutplasma, zur Behandlung der Hyperphosphatämie im Rahmen einer Nierenersatztherapie.

3. Silikathaltiges Tonmineral zur Verwendung nach Anspruch 1 oder 2, **dadurch gekennzeichnet, dass** das Tonmineral mit zweiwertigen Kationen, insbesondere Magnesium-Ionen angereichert ist.

4. Silikathaltiges Tonmineral zur Verwendung nach Anspruch 3, **dadurch gekennzeichnet, dass** die Anreicherung des Tonmineral mit Magnesium-Ionen unter Verwendung von einem Magnesiumsalz, insbesondere Magnesiumchlorid oder Magnesiumsulfat, mit einem Massenanteil zwischen 30 und 70%, bevorzugt 45 und 60% erfolgt.

5. Silikathaltiges Tonmineral zur Verwendung nach einem der vorhergehenden Ansprüche, **dadurch gekennzeichnet, dass** das Tonmineral eine mittlere Teilchengröße von 0,3 bis 0,8 µm, insbesondere bevorzugt von 0,4 bis 0,5 µm aufweist.

6. Silikathaltiges Tonmineral zur Verwendung nach einem der vorhergehenden Ansprüche, **dadurch gekennzeichnet, dass** das Tonmineral mindestens ein Wechsellagerungstonmineral, insbesondere bestehend aus Montmorillonit und Illit/Muskovit umfasst.

7. Silikathaltiges Tonmineral zur Verwendung nach einem der vorhergehenden Ansprüche, **dadurch gekennzeichnet, dass** das Tonmineral als Wechsellagerungstonmineral die Tonminerale Montmorillonit und Illit/Muskovit zu jeweils mindestens 50 Gew% umfasst.

8. Silikathaltiges Tonmineral zur Verwendung nach einem der vorhergehenden Ansprüche, **dadurch gekennzeichnet, dass** weitere Tonminerale wie Kaolinite und Chlorite enthalten sind.

9. Silikathaltiges Tonmineral zur Verwendung nach einem der vorhergehenden Ansprüche, **dadurch gekennzeichnet, dass** das Tonmineral ein Fe²⁺ / Fe³⁺ - Verhältnis zwischen 0,3 und 1, 0, bevorzugt 0,45 und 1,0 aufweist.

10. Silikathaltiges Tonmineral zur Verwendung nach einem der vorhergehenden Ansprüche, **dadurch gekennzeichnet, dass** das Tonmineral 50-60 Gew% Montmorillonit-Muskovit-WL, 15-25 Gew% Illit/Muskovit, 5-9 Gew% Kaolinit/Chlorit, 10-20 Gew% Quarz, 1-2 Gew% Calcit, 0,9-1,5 Gew% Dolomit, 0,9-1,9 Gew% Feldspat, 0,9-2,0 Gew% Pyrit und 0,6-1,0 Gips umfasst.

11. Silikathaltiges Tonmineral zur Verwendung nach einem der vorhergehenden Ansprüche, **dadurch gekennzeichnet, dass** das Tonmineral oral eingenommen wird.

12. Silikathaltiges Tonmineral zur Verwendung nach einem der vorhergehenden Ansprüche, **dadurch gekennzeichnet, dass** das Tonmineral in einer kolloid dispersen Form, insbesondere als 15 % Suspension verwendet wird.

13. Verfahren zur Herstellung eines silikathaltigen Tonminerals nach einem der Ansprüche 1 bis 12 umfassend die Schritte:
a) Zerkleinerung des unbehandelten Tonminerals auf eine mittlere Teilchengröße zwischen 1 und 3 µm, bevorzugt 1 und 2 µm, insbesondere bevorzugt 1,2 und 1,5 µm und Trocknen auf eine Endfeuchte zwischen 0,05 und 1 Masseanteil%, bevorzugt 0,1 und 0,5 Masseanteil%, insbesondere bevorzugt 0,1 Masseanteil und 0,2 Masseanteil%; und
b) thermische Behandlung des zerkleinerten Tonminerals bei einer Temperatur zwischen 400°C und 800°C, bevorzugt zwischen 500°C und 700°C, insbesondere bevorzugt bei 550°C über einen Zeitraum von 60 min bis 240 min, bevorzugt 90 min bis 180 min, insbesondere bevorzugt 120 min.

14. Verfahren nach Anspruch 13, **dadurch gekennzeichnet, dass** in einem weiteren Schritt c) das thermisch behandelten Tonminerals in einer Magnesiumionen- enthaltenden Lösung unter Ausbildung einer kolloidalen Lösung dispergiert wird.

15. Verfahren nach Anspruch 14, **dadurch gekennzeichnet, dass** in einem weiteren Schritt d) die kolloidale Lösung auf eine mittlere Teilchengröße von 0,1 bis 1,5 µm, bevorzugt von 0,3 bis 0,8 µm, insbesondere bevorzugt von 0,4 bis 0,5 µm vermahlen wird.

## Claims

1. Silicate-containing clay mineral for use as agent for reducing the concentration of inorganic phosphate in body fluids or dialysis fluids for treatment of hyperphosphataemia,
**characterized in that**
the silicate-containing clay mineral has an average particle size of 0.1 to 1.5 µm, and **in that** the clay mineral, prior to use, is treated thermally at a temperature between 400°C and 800°C, preferably between 500°C and 700°C, especially preferably at 550°C, over a period of 60 min to 240 min, preferably 90 min to 180 min, especially preferably 120 min.

2. Silicate-containing clay mineral for use according to Claim 1 for reduction of the concentration of inorganic phosphate in blood plasma, for treatment of hyperphosphataemia in the context of renal replacement therapy.

3. Silicate-containing clay mineral for use according to Claim 1 or 2, **characterized in that** the clay mineral has been enriched with divalent cations, especially magnesium ions.

4. Silicate-containing clay mineral for use according to Claim 3, **characterized in that** the enrichment of the clay mineral with magnesium ions is effected using a magnesium salt, especially magnesium chloride or magnesium sulfate, with a proportion by mass between 30% and 70%, preferably 45% and 60%.

5. Silicate-containing clay mineral for use according to any of the preceding claims, **characterized in that** the clay mineral has an average particle size of 0.3 to 0.8 µm, especially preferably of 0.4 to 0.5 µm.

6. Silicate-containing clay mineral for use according to any of the preceding claims, **characterized in that** the clay mineral comprises at least one clay mineral with alternating layers, especially consisting of montmorillonite and illite/muscovite.

7. Silicate-containing clay mineral for use according to any of the preceding claims, **characterized in that** the clay mineral comprises, as clay mineral with alternating layers, the clay minerals montmorillonite and illite/muscovite each to an extent of at least 50% by weight.

8. Silicate-containing clay mineral for use according to any of the preceding claims, **characterized in that** further clay minerals such as kaolinites and chlorites are present.

9. Silicate-containing clay mineral for use according to any of the preceding claims, **characterized in that** the clay mineral has an Fe²⁺/Fe³⁺ ratio between 0.3 and 1.0, preferably 0.45 and 1.0.

10. Silicate-containing clay mineral for use according to any of the preceding claims, **characterized in that** the clay mineral comprises 50-60% by weight of montmorillonite-muscovite with alternating layers, 15-25% by weight of illite/muscovite, 5-9% by weight of kaolinite/chlorite, 10-20% by weight of quartz, 1-2% by weight of calcite, 0.9-1.5% by weight of dolomite, 0.9-1.9% by weight of feldspar, 0.9-2.0% by weight of pyrite and 0.6-1.0 gypsum.

11. Silicate-containing clay mineral for use according to any of the preceding claims, **characterized in that** the clay mineral is taken orally.

12. Silicate-containing clay mineral for use according to any of the preceding claims, **characterized in that** the clay mineral is used in a colloidally dispersed form, especially as a 15% suspension.

13. Process for producing a silicate-containing clay mineral according to any of Claims 1 to 12, comprising the steps of:
a) comminuting the untreated clay mineral to an average particle size between 1 and 3 µm, preferably 1 and 2 µm, especially preferably 1.2 and 1.5 µm, and drying it to a final moisture content between 0.05% and 1% by mass, preferably 0.1% and 0.5% by mass, especially preferably 0.1% by mass and 0.2% by mass; and
b) subjecting the comminuted clay material to thermal treatment at a temperature between 400°C and 800°C, preferably between 500°C and 700°C, especially preferably at 550°C, over a period of 60 min to 240 min, preferably 90 min to 180 min, especially preferably 120 min.

14. Process according to Claim 13, **characterized in that**, in a further step c), the clay mineral that has been subjected to thermal treatment is dispersed in a solution containing magnesium ions to form a colloidal solution.

15. Process according to Claim 14, **characterized in that**, in a further step d), the colloidal solution is ground to an average particle size of 0.1 to 1.5 µm, preferably of 0.3 to 0.8 µm, especially preferably of 0.4 to 0.5 µm.

## Revendications

1. Minéral argileux contenant du silicate pour utilisation en tant que moyen de réduction de la concentration de phosphate inorganique dans des fluides corporels ou des fluides de dialyse pour le traitement d'une hyperphosphatémie,
**caractérisé en ce que**
le minéral argileux contenant du silicate présente une taille moyenne de particule de 0,1 à 1,5 µm, et **en ce que** le minéral argileux est traité thermiquement avant l'utilisation, à une température entre 400 °C et 800 °C, de préférence entre 500 °C et 700 °C, de manière particulièrement préférée à 550 °C sur une durée de 60 min à 240 min, de préférence de 90 min à 180 min, de manière particulièrement préférée de 120 min.

2. Minéral argileux contenant du silicate pour utilisation selon la revendication 1, pour la réduction de la concentration de phosphate inorganique dans le plasma sanguin, pour le traitement de l'hyperphosphatémie dans le cadre d'une thérapie de substitution rénale.

3. Minéral argileux contenant du silicate pour utilisation selon la revendication 1 ou 2, **caractérisé en ce que** le minéral argileux est enrichi en cations divalents, en particulier en ions magnésium.

4. Minéral argileux contenant du silicate pour utilisation selon la revendication 3, **caractérisé en ce que** l'enrichissement du minéral argileux en ions magnésium s'effectue en utilisant un sel de magnésium, en particulier du chlorure de magnésium ou du sulfate de magnésium, avec une proportion en masse entre 30 et 70 %, de préférence 45 et 60 %.

5. Minéral argileux contenant du silicate pour utilisation selon l'une quelconque des revendications précédentes, **caractérisé en ce que** le minéral argileux présente une taille moyenne de particule de 0,3 à 0,8 µm, de manière particulièrement préférée, de 0,4 à 0,5 µm.

6. Minéral argileux contenant du silicate pour utilisation selon l'une quelconque des revendications précédentes, **caractérisé en ce que** le minéral argileux comprend au moins un minéral argileux en feuillets alternés, en particulier constitué de montmorillonite et d'illite/muscovite.

7. Minéral argileux contenant du silicate pour utilisation selon l'une quelconque des revendications précédentes, **caractérisé en ce que** le minéral argileux comprend en tant que minéral argileux en feuillets alternés, les minéraux argileux montmorillonite et illite/muscovite respectivement en quantité d'au moins 50 % en poids.

8. Minéral argileux contenant du silicate pour utilisation selon l'une quelconque des revendications précédentes, **caractérisé en ce que** d'autres minéraux argileux tels que la kaolinite et la chlorite sont présents.

9. Minéral argileux contenant du silicate pour utilisation selon l'une quelconque des revendications précédentes, **caractérisé en ce que** le minéral argileux présente un rapport de Fe²⁺ / Fe³⁺ entre 0,3 et 1,0, de préférence 0,45 et 1,0.

10. Minéral argileux contenant du silicate pour utilisation selon l'une quelconque des revendications précédentes, **caractérisé en ce que** le minéral argileux comprend 50 à 60 % en poids de feuillets alternés de montmorillonite-muscovite, 15 à 25 % en poids d'illite/muscovite, 5 à 9 % en poids de kaolinite/chlorite, 10 à 20 % en poids de quartz, 1 à 2 % en poids de calcite, 0,9 à 1,5 % en poids de dolomite, 0,9 à 1,9 % en poids de feldspath, 0,9 à 2,0 % en poids de pyrite et 0,6 à 1,0 de gypse.

11. Minéral argileux contenant du silicate pour utilisation selon l'une quelconque des revendications précédentes, **caractérisé en ce que** le minéral argileux est pris par voie orale.

12. Minéral argileux contenant du silicate pour utilisation selon l'une quelconque des revendications précédentes, **caractérisé en ce que** le minéral argileux est utilisé sous une forme colloïdale dispersée, en particulier en suspension à 15 %.

13. Procédé de production d'un minéral argileux contenant du silicate selon l'une quelconque des revendications 1 à 12 comprenant les étapes :
a) de réduction du minéral argileux non traité à une taille moyenne de particule entre 1 et 3 µm, de préférence entre 1 et 2 µm, de manière particulièrement préférée entre 1,2 et 1,5 µm, et de séchage à une humidité finale entre 0,05 et 1 % en masse, de préférence entre 0,1 et 0,5 % en masse, de manière particulièrement préférée entre 0,1 % en masse et 0,2 % en masse ; et
b) de traitement thermique du minéral argileux réduit à une température entre 400 °C et 800 °C, de préférence entre 500 °C et 700 °C, de manière particulièrement préférée, à 550 °C sur une durée de 60 min à 240 min, de préférence de 90 min à 180 min, de manière particulièrement préférée, de 120 min.

14. Procédé selon la revendication 13, **caractérisé en ce que** dans une autre étape c) le minéral argileux traité thermiquement est dispersé dans une solution contenant des ions magnésium en formant une solution colloïdale.

15. Procédé selon la revendication 14, **caractérisé en ce que** dans une autre étape d) la solution colloïdale est broyée à une taille moyenne de particule de 0,1 à 1,5 µm, de préférence de 0,3 à 0,8 µm, de manière particulièrement préférée, de 0,4 à 0,5 µm.
